# EUROPEAN PATENT APPLICATION

(11) **EP 1 649 855 A1**
(43) Date of publication of application: **26.04.2006**
(21) Application number: 04747897.9
(22) Date of filing: 16.07.2004
(51) Int. Cl.: A61K 31/381, A61K 31/18, A61K 45/00, A61P 13/08, A61P 13/10, A61P 43/00

(54) **MEDICINAL COMPOSITION**

(30) Priority: 16.07.2003 JP 2003197661
(71) Applicant: KYOWA HAKKO KOGYO CO., LTD., Chiyoda-ku, Tokyo 100-8185 (JP)
(72) Inventor: YAMAGATA, Tsuyoshi Kyowa Hakko Kogyo Co., Ltd., Shizuoka 411-8731 (JP); SHIRAKURA, Shiro Kyowa Hakko Kogyo Co., Ltd., Shizuoka 411-8731 (JP)
(74) Representative: Vossius & Partner
(86) International application number: PCT/JP2004/010533
(87) International publication number: WO 2005/007155

(57) **Abstract**

The present invention provides a pharmaceutical composition which is useful in the treatment for bladder irritative symptoms accompanied by benign prostatic hyperplasia and the like, and comprises 3,3,3-trifluoro-2-hydroxy-2-methyl-N-(5,5,10-trioxo-4,10-dihydrothieno[3,2-c] [1]benzothiepin-9-yl)propanamide or a pharmaceutically acceptable salt thereof, and an α₁-adrenoceptor antagonist.

## Description

### Technical Field

The present invention relates to a pharmaceutical composition which comprises 3,3,3-trifluoro-2-hydroxy-2-methyl-N-(5,5,10-trioxo-4,10-dihydrothieno[3,2-c][1]benzothiepin-9-yl)propanamide (hereinafter referred to as Compound (I), if desired) or a pharmaceutically acceptable salt thereof, and an α₁-adrenoceptor antagonist.

### Background Art

Benign prostatic hyperplasia is benign adenoma derived from a transitional zone of the prostate that is present surrounding the urethra. Patients with benign prostatic hyperplasia complain of bladder outlet obstructive symptoms and/or bladder irritative symptoms. Examples of bladder outlet obstructive symptoms include a delayed initiation of micturition, straining, a weak urinary stream, an intermittent urinary stream, dribbling after urination, prolongation of voiding time and overflow incontinence. Examples of bladder irritative symptoms include urinary frequency at daytime or nighttime, urinary urgency, a feeling of incomplete emptying, and a reduced voided volume per micturition. The pathogenesis of theses symptoms underlies functional and mechanical obstruction in urethras caused by hypertrophied prostate.

Today, the treatments for benign prostatic hyperplasia are surgical operation such as transurethral resection of prostate, and medication of typically an α₁₋adrenoceptor antagonist. The surgical operation for treatment of benign prostatic hyperplasia has some problems: the surgical application is limited since most patients are elderly; the cost of operation is expensive; and the continuation or recurrence of obstructive and irritative symptoms are observed even after surgery. In general, the pharmacological treatment may be mainly used in elderly patients, or patients with mild or moderate benign prostatic hyperplasia.

Activation of a sympathetic nervous system participates in the urethral obstruction accompanied by benign prostatic hyperplasia. Noradrenaline released from the terminal of sympathetic nerves acts to constrict the smooth muscles of a prostate and a urethra, thereby increasing the urethral resistance to decrease the urine flow on urination.

Noradrenaline released from a central nervous system, a peripheral nervous system and a glandular system shows various physiological activities in vivo, and a blocker of α₁-adrenoceptor, one of the receptors of adrenaline, is used for treatment of benign prostatic hyperplasia. In general, however, an α₁-adrenoceptor antagonist is insufficient for ameliorating bladder irritative symptoms though it may be moderately effective for the obstructive symptoms of benign prostatic hyperplasia.

The increase in the urethral resistance due to functional obstruction and mechanical obstruction may further cause secondary histological and functional changes in a detrusor, a sensory nervous system and an autonomic nervous system, and induce complicated disease conditions of bladder irritative symptoms and obstructive symptoms. Associated with such functional changes in the detrusor and nervous systems thereof, patients with benign prostatic hyperplasia often show idiopathic detrusor overactivity.

Various potassium channels is present on sensory nerves and detrusor muscles of urinary bladder, and act to control nervous excitation and detrusor contraction *[The Journal of Physiology,* Vol. 494, No. 1, pp. 1-16 (1996); *Current Drug Targets,* Vol. 2, No. 1, pp. 1-20 (2001); Acta *Physiologica Scandinavica,* Vol. 173, No. 3, pp. 323-333 (2001)]. Hyperexcitability of sensory neurons and increases in detrusor contractions are involved in bladder irritative symptoms accompanied by benign prostatic hyperplasia, and also participate in the development of detrusor overactivity.

Heretofore, it is known that Compound (I) or a pharmaceutically acceptable salt thereof is effective in the treatment of urinary incontinence (WO98/46587), and that Compound (I) or a pharmaceutically acceptable salt thereof has a A-type potassium channel opening activity and is effective in the treatment of bladder irritative symptoms accompanied by benign prostatic hyperplasia (WO02/078633 and WO02/078712).

### Disclosure of the Invention

An object of the present invention is to provide a pharmaceutical composition which comprises Compound (I) or a pharmaceutically acceptable salt thereof and an α₁₋adrenoceptor antagonist, or the like.

The present invention relates to the following (1) to (21):
(1) A pharmaceutical composition which comprises (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 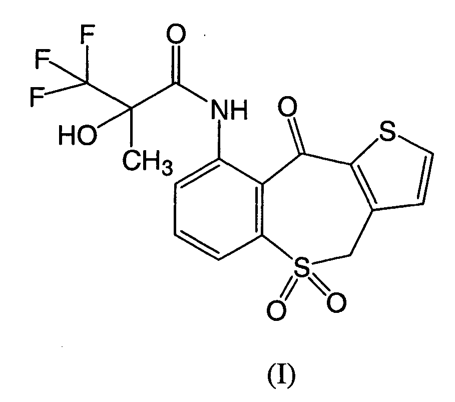 or a pharmaceutically acceptable salt thereof, and (b) an α₁-adrenoceptor antagonist.
(2) The pharmaceutical composition according to (1), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia) : 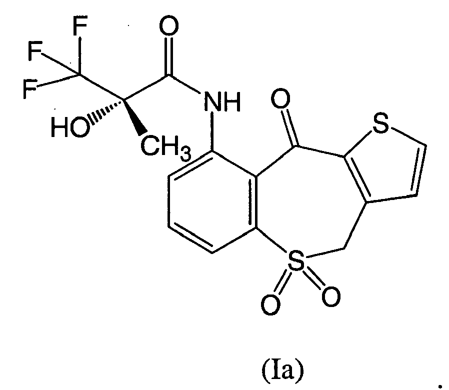
(3) The pharmaceutical composition according to (1) or (2), wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(4) A therapeutic agent for bladder irritative symptoms accompanied by benign prostatic hyperplasia, which comprises, as active ingredients, (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 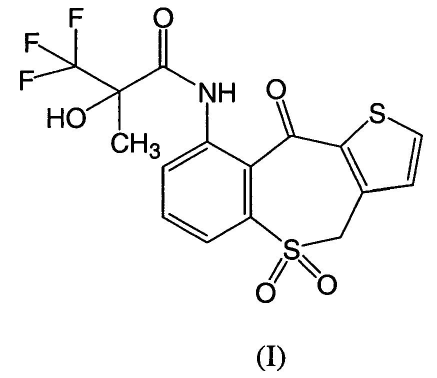 or a pharmaceutically acceptable salt thereof, and (b) an α₁-adrenoceptor antagonist, which may be administered together or separately at an interval.
(5) The therapeutic agent for bladder irritative symptoms accompanied by benign prostatic hyperplasia according to (4), wherein 3,3,3-trifluoro-2-hydroxy-2-methyl-N-(5,5,10-trioxo-4,10-dihydrothieno[3,2-c] [1]benzothiepin-9-yl)propanamide is (S)-(+)-3,3,3-trifluoro-2-hydroxy-2-methyl-N-(5,5,10-trioxo-4,10-dihydrothieno[3,2-c] [1]benzothiepin-9-yl)propanamide represented by Formula (Ia): 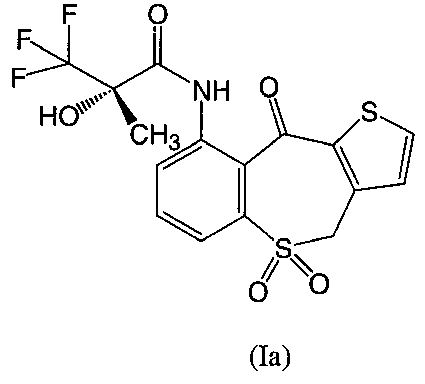
(6) The therapeutic agent for bladder irritative symptoms accompanied by benign prostatic hyperplasia according to (4) or (5), wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(7) A kit which comprises (a) a first component containing 3,3,3-trifluoro-2-hydroxy-2-methyl-N-(5,5,10-trioxo-4,10-dihydrothieno[3,2-c] [1]benzothiepin-9-yl)propanamide represented by Formula (I): 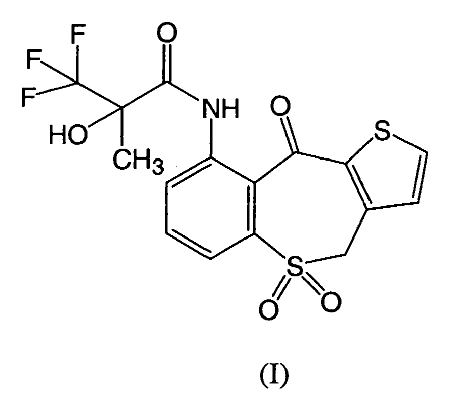 or a pharmaceutically acceptable salt thereof, and (b) a second component containing an α₁-adrenoceptor antagonist.
(8) The kit according to (7), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 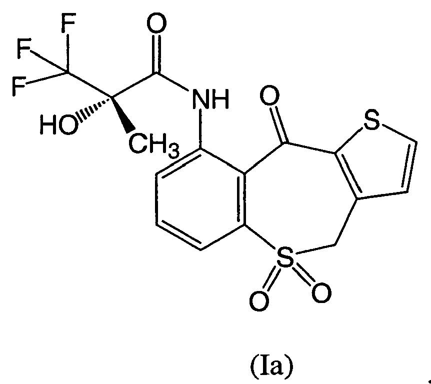
(9) The kit according to (7) or (8), wherein the α₁₋adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(10) A kit for treating bladder irritative symptoms accompanied by benign prostatic hyperplasia, which comprises (a) a first component containing 3,3,3-trifluoro-2-hydroxy-2-methyl-N-(5,5,10-trioxo-4,10-dihydrothieno[3,2-c][1]benzothiepin-9-yl)propanamide represented by Formula (I) : 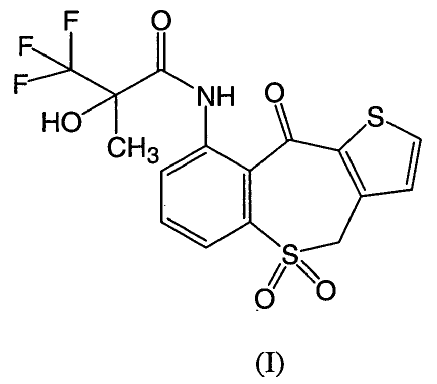 or a pharmaceutically acceptable salt thereof, and (b) a second component containing an α₁-adrenoceptor antagonist.
(11) The kit for treating bladder irritative symptoms accompanied by benign prostatic hyperplasia according to (10), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 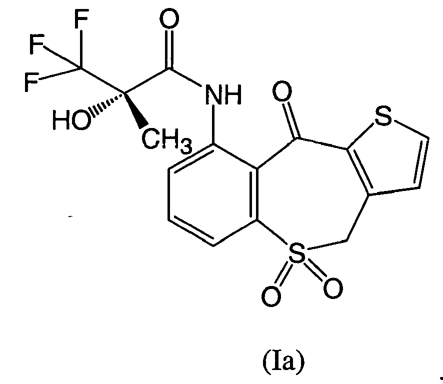
(12) The kit for treating bladder irritative symptoms accompanied by benign prostatic hyperplasia according to (10) or (11), wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(13) 3,3,3-Trifluoro-2-hydroxy-2-methyl-N-(5,5,10-trioxo-4,10-dihydrothieno [3,2-c] [1] benzothiepin-9-yl) propanamide represented by Formula (I): 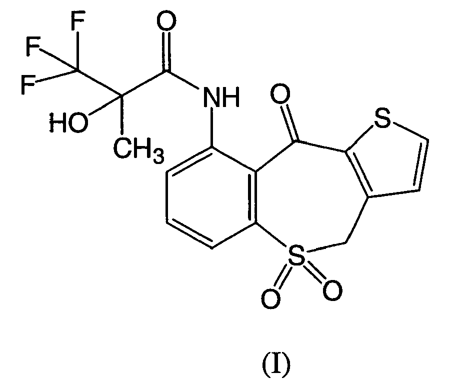 or a pharmaceutically acceptable salt thereof, which may be administered together or separately at an interval with an α₁-adrenoceptor antagonist.
(14) 3,3,3-Trifluoro-2-hydroxy-2-methyl-N-(5,5,10-trioxo-4,10-dihydrothieno[3,2-c] [1]benzothiepin-9-yl)propanamide or a pharmaceutically acceptable salt thereof according to (13), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 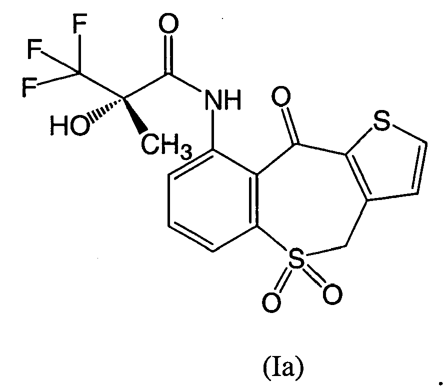
(15) 3,3,3-Trifluoro-2-hydroxy-2-methyl-N-(5,5,10-trioxo-4,10-dihydrothieno[3,2-c] [1]benzothiepin-9-yl)propanamide or a pharmaceutically acceptable salt thereof according to (13) or (14), wherein the α₁₋adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(16) A pharmaceutical composition which comprises, as an active ingredient, 3,3,3-trifluoro-2-hydroxy-2-methyl-N-(5,5,10-trioxo-4,10-dihydrothieno[3,2-c][1]benzothiepin-9-yl)propanamide represented by Formula (I): 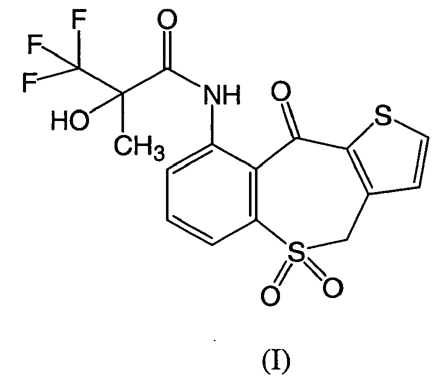 or a pharmaceutically acceptable salt thereof, which may be administered together or separately at an interval with an α₁-adrenoceptor antagonist.
(17) The pharmaceutical composition according to (16), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 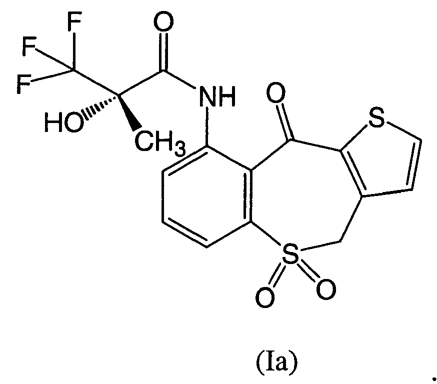
(18) The pharmaceutical composition according to (16) or (17), wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
(19) A method for treating bladder irritative symptoms accompanied by benign prostatic hyperplasia, which comprises administering (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 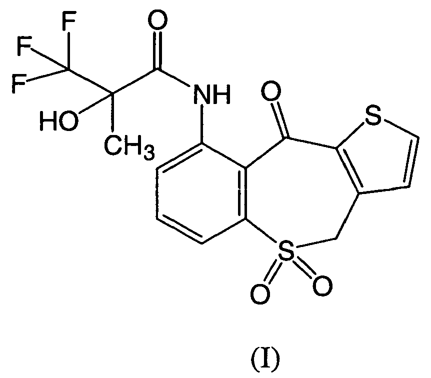 or a pharmaceutically acceptable salt thereof, and (b) an α₁-adrenoceptor antagonist, which may be administered together or separately at an interval.
(20) The method for treating bladder irritative symptoms accompanied by benign prostatic hyperplasia according to (19), wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia): 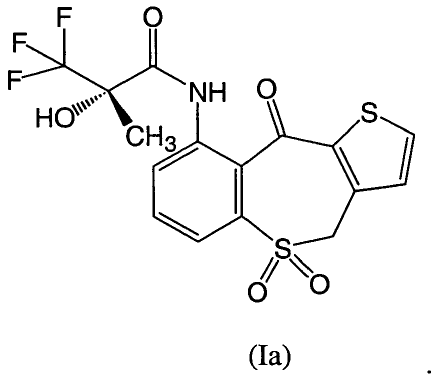
(21) The method for treating bladder irritative symptoms accompanied by benign prostatic hyperplasia according to (19) or (20), wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

The pharmaceutically acceptable salt of Compound (I) includes, for example, pharmaceutically acceptable acid addition salts, metal salts, ammonium salts, organic amine addition salts, amino acid addition salts and the like.

The pharmaceutically acceptable acid addition salts of Compound (I) include inorganic acid salts such as hydrochlorides, sulfates, hydrobromides, nitrates and phosphates; and organic acid salts such as acetates, mesylates, succinates, maleates, fumarates, citrates and tartrates. The pharmaceutically acceptable metal salts include alkali metal salts such as sodium salts and potassium salts; alkaline earth metal salts such as magnesium salts and calcium salts; and aluminum salts and zinc salts. The pharmaceutically acceptable ammonium salts include ammonium salts, tetramethylammonium salts and the like. The pharmaceutically acceptable organic amine addition salts include salts with morpholine, piperidine and the like. The pharmaceutically acceptable amino acid addition salts include addition salts with glycine, phenylalanine, lysine, aspartic acid, glutamic acid and the like.

A production method of Compound (I) is described.

Compound (I) can be produced according to the method described in WO98/4658 or a similar method thereof.

There may be stereoisomers (for example, tautomers, enantiomers and the like) for Compound (I). All possible isomers and mixtures thereof including above-mentioned stereoisomers can be used as the pharmaceutical composition, the therapeutic agent for bladder irritative symptoms accompanied by benign prostatic hyperplasia, the kit, the kit for treating bladder irritative symptoms accompanied by benign prostatic hyperplasia and the method for treating bladder irritative symptoms accompanied by benign prostatic hyperplasia of the present invention. Compound (I) of the present invention includes all possible isomers and mixtures thereof including above-mentioned stereoisomers.

To obtain a salt of Compound (I), it may be purified as it is when it is produced in the form of the salt, and when it is produced in the form of a free form, it may be dissolved or suspended in a suitable solvent, and added with an acid or a base, followed by isolation and purification.

Compound (I) and pharmaceutically acceptable salts thereof may exist in the form of adducts with water or various solvents, and such adducts can also be used as the pharmaceutical composition, the therapeutic agent for bladder irritative symptoms accompanied by benign prostatic hyperplasia, the kit, the kit for treating bladder irritative symptoms accompanied by benign prostatic hyperplasia and the method for treating bladder irritative symptoms accompanied by benign prostatic hyperplasia of the present invention, and such adducts are included in Compound (I) and pharmaceutically acceptable salts thereof of the present invention.

The α₁-adrenoceptor antagonist includes tamsulosin, prazosin, terazosin, urapidil, doxazosin, alfzosin, naftopidil, maftopidil, abanoxil and indolamin, as well as stereoisomers (for example, enantiomers and the like) thereof, pharmaceutically acceptable salts thereof and hydrates thereof. One or more of these may be used herein either singly or as combined. The pharmaceutically acceptable salts of the compounds are, for example, the salts mentioned hereinabove for the pharmaceutically acceptable salts of Compound (I).

Compound (I) or a pharmaceutically acceptable salt thereof and the α₁-adrenoceptor antagonist used in the pharmaceutical composition or the therapeutic agent for bladder irritative symptoms accompanied by benign prostatic hyperplasia of the present invention may be administered alone or in combination as preparations containing their active ingredients. Particularly, a combination of two or more preparations is preferable. When the preparations are used or administered in combination, they may be used or administered together or separately at an interval.

The dose ratio (weight/weight) of Compound (I) or a pharmaceutically acceptable salt thereof to the α₁₋adrenoceptor antagonist may be suitably determined, depending on the combination with the α₁-adrenoceptor antagonist to be used and the efficacy of the α₁₋adrenoceptor antagonist. For example, the specific ratio is from 1/500 (Compound (I) or a pharmaceutically acceptable salt thereof/α₁-adrenoceptor antagonist) to 5000/1, more preferably from 1/300 to 1000/1, even more preferably from 1/200 to 500/1, still more preferably from 1/100 to 100/1.

When the preparations are administered in combination, for example, for example, (a) a first component comprising Compound (I) or a pharmaceutically acceptable salt thereof, and (b) a second component comprising an α₁-adrenoceptor antagonist are separately prepared as described above, and made into a kit. By utilizing such a kit, each component can be administered together or separately at an interval to one subject by the same route or different routes.

The kit comprises two or more containers (for example, vials, bags) and the contents. The material and the shape of the containers are not limited, but the containers must prevent the contents, i.e. the components, from degrading due to external temperature or light during the storage, and should be made from a material that does not elute its chemical constituents. The first component and the second component are administerable dosage forms so as to be administered through different routes (for example, tubes) or the same route. Specifically mentioned are a kit of tablets, injections, and the like.

The method for treating bladder irritative symptoms accompanied by benign prostatic hyperplasia of the present invention may be carried out in the same manner as that for the use or administration of Compound (I) or a pharmaceutically acceptable salt thereof and an α₁₋adrenoceptor antagonist used in the pharmaceutical composition or the therapeutic agent for bladder irritative symptoms accompanied by benign prostatic hyperplasia described above. That is, Compound (I) or a pharmaceutically acceptable salt thereof and an α₁₋adrenoceptor antagonist are formulated into preparations containing the respective active ingredients therein, and for example, they are administered as single preparation or as a combination of preparations, preferably, they are administered as a combination of two or more preparations. When preparations are administered in combinnation, they may be administered together or separately at an interval, and the kit described above may be used for administration.

The efficacy of treatment of bladder irritative symptoms accompanied by benign prostatic hyperplasia by the combined administration of Compound (I) or a pharmaceutically acceptable salt thereof and an α₁₋adrenoceptor antagonist will be described specifically with reference to Test Example. In the following Test Example, (S)-(+)-3,3,3-trifluoro-2-hydroxy-2-methyl-N-(5,5,10-trioxo-4,10-dihydrothieno[3,2-c][1]benzothiepin-9-yl)propanamide (hereinafter referred to as Compound (Ia), if desired) was used as Compound (I).

### Test Example: Inhibitory Activity on Detrusor Overactivity

The experiment was carried out according to the method by Malmgren, et al. [J. Urol., Vol. 142, pp. 1134-1138 (1989)].

Female SD rats of 8 to 9 weeks of age (supplied by Charles River Japan) were used in the test. Five to seven animals of these rats were housed in metal cages and given commercially available chow and water *ad libtum,* in an animal room maintained at room temperature from 19°C to 25°C and humidity from 30% to 70% under illumination for 12 hours (7:00 a.m. - 7:00 p.m.) per day.

The rats were subjected to operation of partial urethral obstruction. Each animal was anesthetized by intraperitoneal administration of 50 mg/kg pentobarbital sodium (Tokyo Kasei), and the midline incision of the skin and muscle was made in the lower abdomen. A polyethylene tube (PE-20, Becton Dickinson) was inserted into urethra from the urethral orifice to the bladder neck. After the proximal urethra was separated from connective tissues and ligated using double suture, the polyethylene tube was removed to induce partial urethral obstruction. The incised part was sutured with a surgical silk thread. An antibiotic ampicillin (Sigma) was intramusclarly injected (150 mg/kg, once a day, for 3 days).

Six weeks after the operation of the partial urethral obstruction, the rats with bladder hypertrophy were subjected to intravesical implantation of catheter. Under anesthesia with pentobarbital sodium, the bladder was exposed by an abdominal midline incision. A polyethylene tube (PE-50, Becton Dickinson), one end of which was blunted not to damage the bladder tissue, was filled with a physiological saline (Otsuka Pharmaceutical Factory) and inserted into the bladder via the bladder apex. The intravesical catheter was fixed with a surgical silk suture. The other end of the catheter was tunneled subcutaneously, drawn out from the dorsal neck, plugged and then fixed to the skin with a suture.

Four to seven days after the surgery for implantation of intravesical catheter, a cystometry test was performed. The rats were housed in a Bollman cage (Natsume Seisakusho) and a three-way stopcock was connected to the intravesical catheter, one end of the stopcock was connected to a pressure transducer (Nihon Kohden) and the other end was connected to a 50-mL syringe (Terumo) set in an infusion pump (KD Scientific) for physiological saline infusion. The intravesical pressure was measured with a polygraph system (RMP-6008, Nihon Kohden) via a pressure transducer and a strain pressure amplifier (AP-621G, Nihon Kohden), and recorded on a thermal array recorder (RTA-1200, Nihon Kohden). Sixty to 90 minutes after the completion of the preparation for the measurement, a room temperature physiological saline was continuously infused into the urinary bladder for 30 minutes at a flow rate of 10 mL/h to confirm the occurrence of micturition contractions and premicturition contractions (detrusor overactivity). Thirty minutes after the cessation of the first saline infusion, the saline was again infused intravesically for 30 minutes, and this period is for determination of the data before drug administration. Compound (Ia) was suspended in a 0.5 w/v (weight/volume) % methyl cellulose solution at a concentration of 1 mg/ml. The suspension was further diluted with a 0.5 w/v % methyl cellulose solution to prepare solution for administration [solution for Compound (Ia) administration] at a concentration of 0.01 mg/ml. The solution was orally administered to the rats at a volume of 1 ml/kg. Tamsulosin hydrochloride was dissolved in a 0.5 w/v % methyl cellulose solution at a concentration of 0.03 mg/ml (solution for tamsulosin administration), and the solution was orally administered to the rats at a volume of 1 ml/kg. For investigating the effect of the combined administration, Compound (Ia) solution and the tamsulosin solution were simultaneously and orally administered to the rats, both at a volume of 1 ml/kg each. The periods of 1, 3 and 5 hours after the administration were used as the measuring time after the administration of the vehicle or drug. During a duration of 15 minutes before and after each measuring time (45 to 75 minutes, 165 to 195 minutes and 285 to 315 minutes after the drug administration), a physiological saline was infused into the bladder of each rat.

Micturition contractions and premicturition contractions were measured as an index of voiding function and detrusor overactivity, respectively. The average of all micturition contraction values observed during each 30-minute measurement period was designated as the amplitude of micturition contraction, and the average of the maximum premicturition contraction between respective micturition contractions were designated as the amplitude of premicturition contractions at each measurement point. The number of premicturition contractions for 2 minutes just before voiding was counted, and this is referred to as frequency of premicturition contractions. The values of both contractions and frequency were read from intravesical pressure waveforms recorded on a chart using a digitizer (KW4620, Graphtec Corporation) controlled by a computer (PC-9801NS/R, NEC), and saved as a DAT-format file or a WJ2-format file. The data files were imported into Excel 2000 (Microsoft). The amplitude and frequency of premicturition contractions, and the amplitude of micturition contractions were expressed as relative values when the values before the drug administration were defined as 100, and the average ± standard error was calculated for each group.

The values (%) of amplitude of premicturition contractions after vehicle or drug administration are shown in Table 1, the values (%) of frequency of premicturition contractions are shown in Table 2, and the values (%) of amplitude of micturition contractions are shown in Table 3.

**Table 1 : Effect of Combination of Compound (la) and Tamsulosin on amplitude of premicturition contractions in rats with bladder hypertrophy**

| | control | Compound (Ia) 0.01 mg/kg, p.o. | Tamsulosin 0.03 mg/kg, p.o. | Compound (Ia) + Tamsulosin |
|---|---|---|---|---|
| Before administration | 100.0 ± 0.0 | 100.0 ± 0.0 | 100.0 ± 0.0 | 100.0 ± 0.0 |
| After 1 hour | 107.1 ± 13.3 | 62.3 ± 7.7** | 62.0 ± 5.2** | 45.9 ± 6.8** |
| After 3 hours | 107.1 ± 12.3 | 62.8 ± 7.6** | 75.2 ± 5.5* | 45.6 ± 6,7***'‡‡ |
| After 5 hours | 113.6 ± 9.6 | 65.6 ± 7.5*** | 88.3 ± 7.2* | 42.9 ± 4.4***,†,‡‡‡ |

| | | | | |
|---|---|---|---|---|
| *P<0.05, **P<0.01, ***P<0.001 (compared with control group) | | | | |
| ^{†}P<0.05 (compared with Compound (Ia) administration group) | | | | |
| ^{‡‡}P<0.01, ^{‡‡‡}P<0.001 (compared with tamsulosin administration group) (n=9-10; Student's t-test or Aspin-Welch test) | | | | |

**Table 2 : Effect of Combination of Compound (I) and Tamsulosin on frequency of premicturition contraction in rats with bladder hypertrophy**

| | control | Compound (Ia) 0.01 mg/kg, p.o. | Tamsulosin 0.03 mg/kg, p.o. | Compound (Ia) + Tamsulosin |
|---|---|---|---|---|
| Before administration | 100.0 ± 0.0 | 100.0 ± 0.0 | 100.0 ± 0.0 | 100.0 ± 0.0 |
| After 1 hour | 107.9 ± 16.5 | 52.3 ± 7.3 ** | 57.8 ± 7.5^{*} | 26.3 ± 5.4^{**,†,‡‡} |
| After 3 hours | 136.4 ± 27.0 | 46.2 ± 8,1^{**} | 73.2 ± 5.3^{*} | 26.0 ± 9.5 ^{**' ‡‡‡} |
| After 5 hours | 103.3 ± 15.3 | 42.4 ± 6.0^{**} | 78.4 ± 7.0 | 20.1 ± 4.0^{**,†,‡‡‡} |

| | | | | |
|---|---|---|---|---|
| *P<0.05, **P<0.01, ***P<0.001 (compared with control group) | | | | |
| ^{t}P<0.05 (compared with Compound (la) administration group) | | | | |
| ^{‡‡}P<0.01, ^{‡‡‡}P<0.001 (compared with tamsulosin administration group) (n=9-10; Student's t-test or Aspin-Welch test) | | | | |

**Table 3 : Effect of Compound (Ia) and Tamsulosin on amplitude of micturition contractions in rats with bladder hypertrophy**

| | control | Compound (Ia) 0.01 mg/kg, p.o. | Tamsulosin 0.03 mg/kg, p.o. | Compound (la) + Tamsulosin |
|---|---|---|---|---|
| Before administration | 100.0 ± 0.0 | 100.0 ± 0.0 | 100.0 ± 0.0 | 100.0 ± 0.0 |
| After 1 hour | 106.5 ± 5.1 | 109.5 ± 2.2 | 108.5 ± 6.4 | 106.3 ± 6.4 |
| After 3 hours | 103.2 ± 2.8 | 105.5 ± 3.8 | 108.1 ± 6.0 | 106.0 ± 3.8 |
| After 5 hours | 100.2 ± 2.6 | 96.7 ± 2.4 | 104.7 ± 6.4 | 102.8 ± 1.6 |

According to the results of Test Example, Compound (Ia) and tamsulosin inhibited premicturition contractions (amplitude and frequency of premicturition contractions). Combined administration of Compound (Ia) and tamsulosin resulted in enhanced inhibition of premicturition contractions (amplitude and frequency of pre-micturition contractions). Administration of Compound (Ia) alone, administration of tamsulosin alone, and combined administration of Compound (Ia) and tamsulosin all did not affect micturition contractions, or that is, they did not have any influence on voiding function itself.

According to the above results, it is thought that a combination of Compound (I) or a pharmaceutically acceptable salt thereof and an α₁-adrenoceptor antagonist may be useful for the treatment of bladder irritative symptoms accompanied by benign prostatic hyperplasia.

As described above, the pharmaceutical composition or the therapeutic agent for bladder irritative symptoms accompanied by benign prostatic hyperplasia of the present invention may be used, administered or produced in a single preparation or a combination of preparations so far as the preparations are formulated so as to contain the respective active ingredients, Compound (I) or a pharmaceutically acceptable salt thereof and an α₁-adrenoceptor antagonist. Preferably, the pharmaceutical composition or the therapeutic agent for bladder irritative symptoms accompanied by benign prostatic hyperplasia has a unit dose form suitable to oral administration such as tablets or capsule, or has a unit dose form suitable to parenteral administration such as injections. When preparations are used or administered as a combination of preparations, they may be used or administered together or separately at an interval.

These preparations may be produced in any ordinary method using any other pharmaceutically acceptable diluent, excipient, disintegrator, lubricant, binder, surfactant, water, physiological saline, vegetable oil solubilizer, isotonizing agent, preservative and antioxidant, in addition to the respective active ingredients.

In preparing tablets and capsules, for example, excipients such as lactose, disintegrators such as starch, lubricants such as magnesium stearate, binders such as hydroxypropyl cellulose, surfactants such as fatty acid ester, and plasticizers such as glycerin can be used according to any ordinary manner.

In preparing injections, for example, carriers such as distilled water, salt solution, glucose solution or a mixture of salt water and glucose solution, solubilizers, isotonizing agents, preservatives, and antioxidants can be used according to any ordinary manner.

When Compound (I) or a pharmaceutically acceptable salt thereof and an α₁-adrenoceptor antagonist are used or administered as a combination of preparations for the object described above, the dose and the administration frequency thereof may vary depending on the dosage form, the age, the body weight and the condition of patients. In general, Compound (I) or a pharmaceutically acceptable salt thereof and an α₁-adrenoceptor antagonist are preferably administered in a dose described below per day:
Compound (I) or a pharmaceutically acceptable salt thereof can be administered orally or parenterally as injection or the like. Its dose may be from 0.01 to 900 mg/60 kg/day, preferably from 0.1 to 200 mg/60 kg/day, per an adult. The dose of an α₁-adrenoceptor antagonist may be from 0.01 to 500 mg/60 kg/day, preferably from 0.2 to 100 mg/60 kg/day, per an adult.

Embodiments of the present invention are described below with reference to the following Examples, which, however do not restrict the scope of the invention.

### Best Modes for Carrying Out the Invention

### Example 1: Tablets (Compound (Ia))

Tablets having the following compositions were prepared according to an ordinary method.

Compound (Ia) (250 g), mannitol (1598.5 g), sodium starch glycolate (100 g), light silicic anhydride (10 g), magnesium stearate (40 g) and yellow iron oxide (1.5 g) were mixed according to an ordinary method. The resulting mixture was compressed using a tableting machine with 8 mm diameter punch and die (Purepress Correct-12, Kikusui Seisakusho) to prepare tablets (containing 25 mg of the active ingredient per tablet).

**Formulation**

| | |
|---|---|
| Compound (Ia) | 25 mg |
| Mannitol | 159.85 mg |
| Sodium starch glycolate | 10 mg |
| Light silicic anhydride | 1 mg |
| Magnesium stearate | 4 mg |
| Yellow iron oxide | 0.15 mg |
| | 200 mg |

### Example 2: Capsules (Compound (Ia))

Capsules having the following compositions were prepared according to an ordinary method.

Compound (Ia) (500 g), lactose (300 g), light silicic anhydride (100 g) and sodium lauryl sulfate (100 g) were mixed according to an ordinary manner. The resulting mixture was encapsulated in hard capsules No. 1 (content: 100 mg/capsule) using a capsule filler (LZ-64, Zanasi) to prepare capsules (containing 50 mg of the active ingredient per capsule).

**Formulation**

| | |
|---|---|
| Compound (Ia) | 50 mg |
| Lactose | 30 mg |
| Light silicic anhydride | 10 mg |
| Sodium lauryl sulfate | 10 mg |
| | 100 mg |

### Example 3: Injection (Compound (Ia))

An injection having the following compositions is prepared according to an ordinary method.

Compound (Ia) (1 g) and D-mannitol (5 g) are added to and mixed with distilled water for injection, and an aqueous hydrochloric acid solution and an aqueous sodium hydroxide solution are added thereto to control pH of the resulting solution to 6. Then, distilled water for injection is added thereto to make the content 1000 ml in total. Under a germ-free condition, 2 ml of the resulting mixture is put into each glass, and an injection was thus obtained (containing 2 mg of the active ingredient per vial) .

**Formulation**

| | |
|---|---|
| Compound (Ia) | 2 mg |
| D-mannitol | 10 mg |
| Aqueous hydrochloric acid solution | proper quantity |
| Aqueous sodium hydroxide solution | proper quantity |
| Distilled water for injection | proper quantity |
| | 2.00 ml |

### Example 4: Tablets (tamsulosin hydrochloride)

Tablets having the following composition are prepared according to an ordinary method.

Tamsulosin hydrochloride (0.4 g), lactose (303.6 g) and potato starch (68 g) are mixed, and a 10% aqueous hydroxypropyl cellulose solution (200 g) is added to the mixture. The resulting mixture is kneaded according to an ordinary manner, then granulated and dried to give granules to be tabletted. 8.0 g of magnesium stearate is added thereto, and using a tabletting machine with 8 mm diameter punch and die (RT-15 Model, Kikusui Seisakusho), the resulting mixture is tabletted to give tablets (containing 0.2 mg of the active ingredient per tablet).

**Formulation**

| | |
|---|---|
| Tamuslosin hydrochloride | 0.2 mg |
| Lactose | 151.8 mg |
| Potato starch | 34 mg |
| Hydroxypropyl cellulose | 10 mg |
| Magnesium stearate | 4 mg |
| | 200 mg |

### Example 5: Tablets (single preparation of Compound (Ia) and tamsulosin hydrochloride)

Tablets having the following composition are prepared according to an ordinary method.

Compound (Ia) (40 g), tamsulosin hydrochloride (0.4 g), lactose (289.6 g) and potato starch (56 g) are mixed, and a 10% aqueous hydroxypropyl cellulose solution (120 g) is added to the mixture. The resulting mixture is kneaded according to an ordinary manner, then granulated and dried to give granules to be tabletted. 2 g of magnesium stearate is added thereto, and using a tabletting machine with 8 mm diameter punch and die (RT-15 Model, Kikusui Seisakusho), the resulting mixture is tabletted to give tablets (containing 20 mg of compound (Ia) and 0.2 mg of tamsulosin hydrochloride per tablet).

**Formulation**

| | |
|---|---|
| Compound (Ia) | 20 mg |
| Tamsulosin hydrochloride | 0.2 mg |
| Lactose | 144.8 mg |
| Potato starch | 28 mg |
| Hydroxypropyl cellulose | 6 mg |
| Magnesium stearate | 1 mg |
| | 200 mg |

### Industrial Applicability

The present invention provides a pharmaceutical composition which comprises 3,3,3-trifluoro-2-hydroxy-2-methyl-N-(5,5,10-trioxo-4,10-dihydrothieno[3,2-c][1]benzothiepin-9-yl)propanamide or a pharmaceutically acceptable salt thereof, and an α₁-adrenoceptor antagonist.

## Claims

1. A pharmaceutical composition which comprises (a) 3,3,3-trifluoro-2-hydroxy-2-methyl-N-(5,5,10-trioxo-4,10-dihydrothieno[3,2-c][1]benzothiepin-9-yl)propanamide represented by Formula (I): 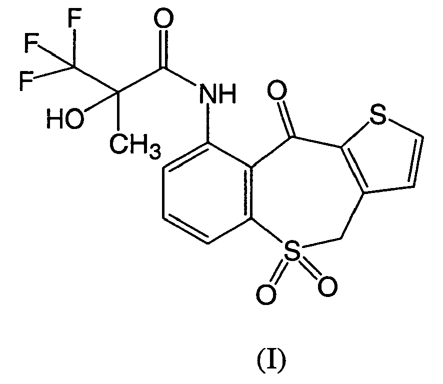 or a pharmaceutically acceptable salt thereof, and (b) an α₁-adrenoceptor antagonist.

2. The pharmaceutical composition according to Claim 1, wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

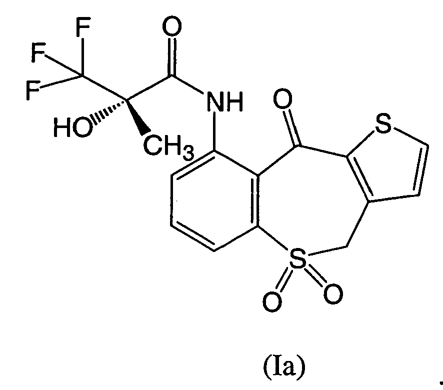

3. The pharmaceutical composition according to Claim 1 or 2, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

4. A therapeutic agent for bladder irritative symptoms accompanied by benign prostatic hyperplasia, which comprises, as active ingredients, (a) 3,3,3-trifluoro-2-hydroxy-2-methyl-N-(5,5,10-trioxo-4,10-dihydrothieno[3,2-c][1]benzothiepin-9-yl)propanamide represented by Formula (I) : 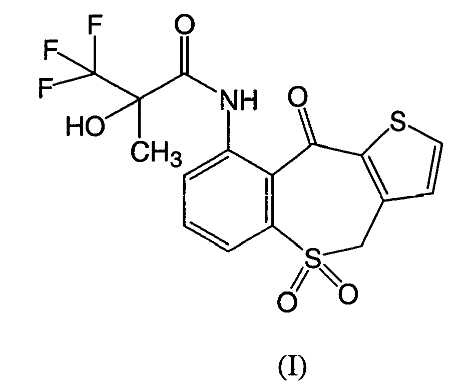 or a pharmaceutically acceptable salt thereof, and (b) an α₁-adrenoceptor antagonist, which may be administered together or separately at an interval.

5. The therapeutic agent for bladder irritative symptoms accompanied by benign prostatic hyperplasia according to Claim 4, wherein 3,3,3-trifluoro-2-hydroxy-2-methyl-N-(5,5,10-trioxo-4,10-dihydrothieno[3,2-c] [1]benzothiepin-9-yl)propanamide is (S)-(+)-3,3,3-trifluoro-2-hydroxy-2-methyl-N-(5,5,10-trioxo-4,10-dihydrothieno[3,2-c] [1]benzothiepin-9-yl)propanamide represented by Formula (Ia):

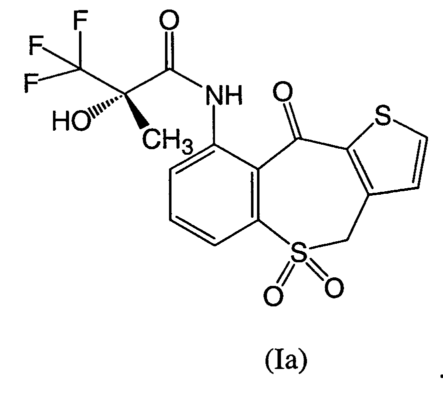

6. The therapeutic agent for bladder irritative symptoms accompanied by benign prostatic hyperplasia according to Claim 4 or 5, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

7. A kit which comprises (a) a first component containing 3,3,3-trifluoro-2-hydroxy-2-methyl-N-(5,5,10-trioxo-4,10-dihydrothieno[3,2-c][1]benzothiepin-9-yl)propanamide represented by Formula (I): 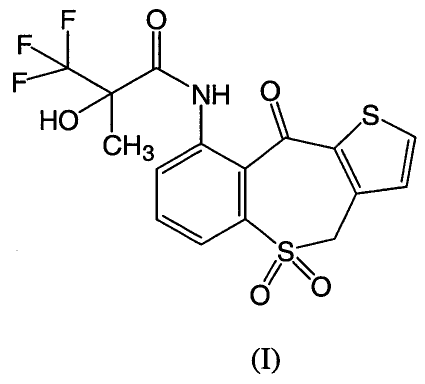 or a pharmaceutically acceptable salt thereof, and (b) a second component containing an α₁-adrenoceptor antagonist.

8. The kit according to Claim 7, wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

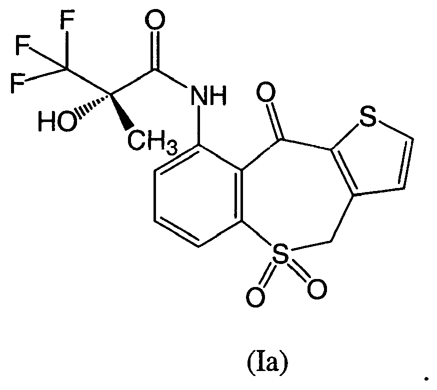

9. The kit according to Claim 7 or 8, wherein the α₁₋adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

10. A kit for treating bladder irritative symptoms accompanied by benign prostatic hyperplasia, which comprises (a) a first component containing 3,3,3-trifluoro-2-hydroxy-2-methyl-N-(5,5,10-trioxo-4,10-dihydrothieno[3,2-c] [1]benzothiepin-9-yl)propanamide represented by Formula (I) : 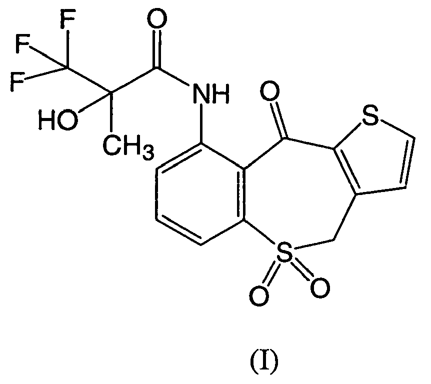 or a pharmaceutically acceptable salt thereof, and (b) a second component containing an α₁-adrenoceptor antagonist.

11. The kit for treating bladder irritative symptoms accompanied by benign prostatic hyperplasia according to Claim 10, wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia) :

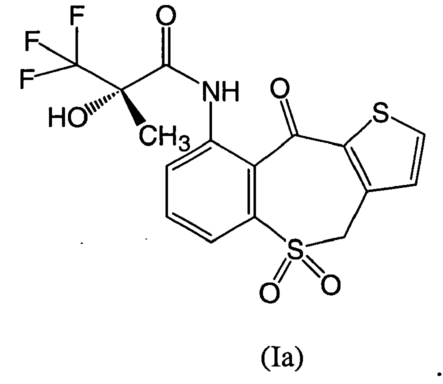

12. The kit for treating bladder irritative symptoms accompanied by benign prostatic hyperplasia according to Claim 10 or 11, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

13. 3,3,3-Trifluoro-2-hydroxy-2-methyl-N-(5,5,10-trioxo-4,10-dihydrothieno[3,2-c] [1]benzothiepin-9-yl)propanamide represented by Formula (I): 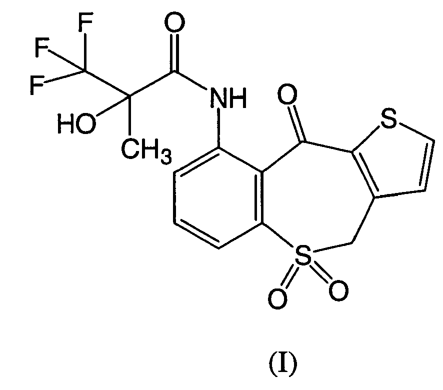 or a pharmaceutically acceptable salt thereof, which may be administered together or separately at an interval with an α₁-adrenoceptor antagonist.

14. 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to Claim 13, wherein 3,3,3-trifluoro-2-hydroxy-2-methyl-N-(5,5,10-trioxo-4,10-dihydrothieno[3,2-c][1]benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

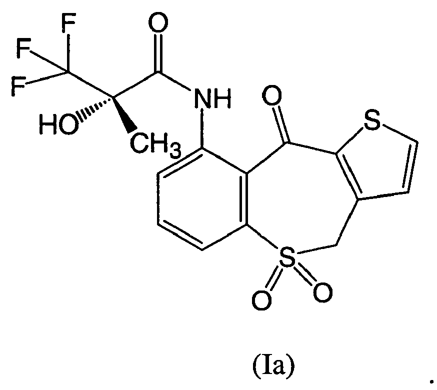

15. 3,3,3-Trifluoro-2-hydroxy-2-methyl-N-(5,5,10-trioxo-4,10-dihydrothieno[3,2-c][1]benzothiepin-9-yl)propanamide or a pharmaceutically acceptable salt thereof according to Claim 13 or 14, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

16. A pharmaceutical composition which comprises, as an active ingredient, 3,3,3-trifluoro-2-hydroxy-2-methyl-N-(5,5,10-trioxo-4,10-dihydrothieno[3,2-c][1]benzothiepin-9-yl)propanamide represented by Formula (I): 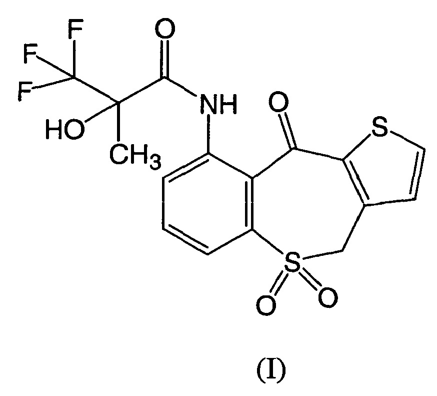 or a pharmaceutically acceptable salt thereof, which may be administered together or separately at an interval with an α₁-adrenoceptor antagonist.

17. The pharmaceutical composition according to Claim 16, wherein 3,3,3-trifluoro-2-hydroxy-2-methyl-N-(5,5,10-trioxo-4,10-dihydrothieno[3,2-c] [1]benzothiepin-9-yl)propanamide is (S)-(+)-3,3,3-trifluoro-2-hydroxy-2-methyl-N-(5,5,10-trioxo-4,10-dihydrothieno[3,2-c] [1]benzothiepin-9-yl)propanamide represented by Formula (Ia) :

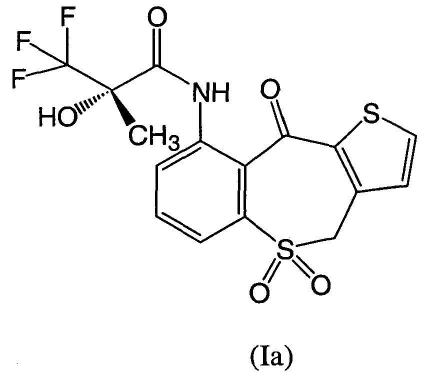

18. The pharmaceutical composition according to Claim 16 or 17, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.

19. A method for treating bladder irritative symptoms accompanied by benign prostatic hyperplasia, which comprises administering (a) 3,3,3-trifluoro-2-hydroxy-2-methyl-N-(5,5,10-trioxo-4,10-dihydrothieno[3,2-c][1]benzothiepin-9-yl)propanamide represented by Formula (I) : 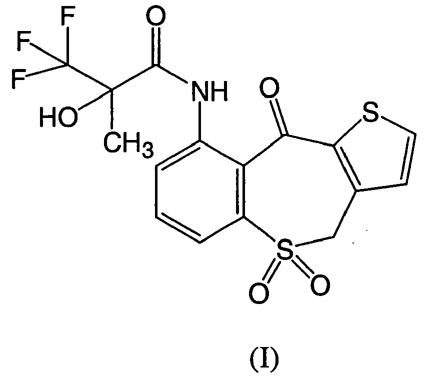 or a pharmaceutically acceptable salt thereof, and (b) an α₁-adrenoceptor antagonist, which may be administered together or separately at an interval.

20. The method for treating bladder irritative symptoms accompanied by benign prostatic hyperplasia according to Claim 19, wherein 3,3,3-trifluoro-2-hydroxy-2-methyl-N-(5,5,10-trioxo-4,10-dihydrothieno[3,2-c] [1] benzothiepin-9-yl)propanamide is (S)-(+)-3,3,3-trifluoro-2-hydroxy-2-methyl-N-(5,5,10-trioxo-4,10-dihydrothieno[3,2-c][1]benzothiepin-9-yl)propanamide represented by Formula (Ia):

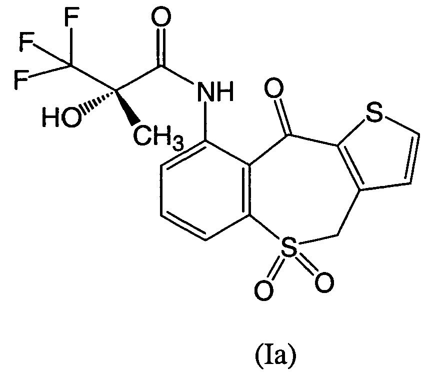

21. The method for treating bladder irritative symptoms accompanied by benign prostatic hyperplasia according to Claim 19 or 20, wherein the α₁-adrenoceptor antagonist is/are selected from any one of or more than one of the following: tamsulosin, prazosin, terazosin, urapidil, doxazosin, alfzosin, naftopidil, maftopidil, abanoxil and indolamin, and pharmaceutically acceptable salts thereof.
